# EUROPEAN PATENT SPECIFICATION

(11) **EP 1 676 536 B1**
(45) Date of publication and mention of the grant of the patent: **21.01.2009**
(21) Application number: 05257904.2
(22) Date of filing: 21.12.2005
(51) Int. Cl.: A61B 17/22

(54) **Systems for removing plaque from a blood vessel**
Gerät zur Entfernung von Plaque aus Blutgefässen
Appareil permettant d'éliminer la plaque déposée sur les vaisseaux sanguins

(30) Priority: 29.12.2004 US 25236
(43) Date of publication of application: 05.07.2006
(73) Proprietor: Nitinol Development Corporation, Fremont, California 94539 (US)
(72) Inventor: Meretei, Attila, Fremont, CA 94538 (US)
(74) Representative: Gover, Richard Paul

(56) References cited:
- WO-A-02/24271
- US-B1- 6 231 496

## Description

The invention generally relates to systems for removing plaque from a blood vessel. More specifically, the invention relates to systems for delivering and manipulating ferrofluids in the bloodstream of the patient to remove plaque from the blood vessel.

Plaque formation begins as fatty streaks or deposits on the inner surface of a blood vessel, such as on the inner surface of arterial walls. Over time, the fat deposits accumulate and grow, narrowing the lumen of the blood vessel and hardening the blood vessel wall due to various depositions occurring within the plaque such as lipids, cholesterol, and calcium salts. The narrowing and hardening of the blood vessels can have dramatic effects on blood pressure and blood flow within the blood vessels.

For example, when blood flow is reduced due to the narrowing of a blood vessel, organs may be deprived of oxygen intended to be carried in greater volume through the blood vessels. If the oxygen supply to the heart muscle is reduced sufficiently, a heart attack can occur. If the oxygen supply to the brain is reduced sufficiently, a stroke can occur. Likewise, where hardening of the blood vessel walls occur due to plaque accumulations, the ability of the blood vessel to distend is reduced resulting in an increase of blood pressure in the blood vessels. As blood pressure increases, the heart has to work harder to pump blood, undesirably causing the heart to enlarge.

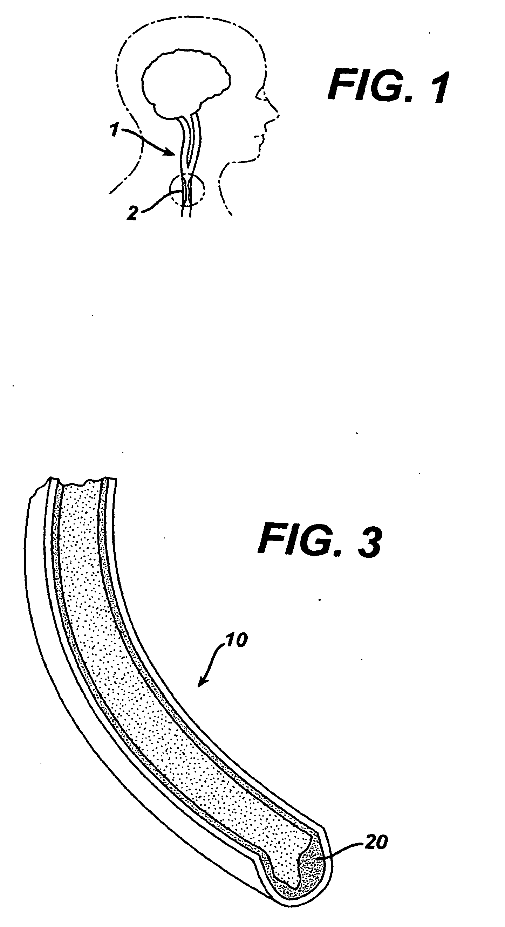

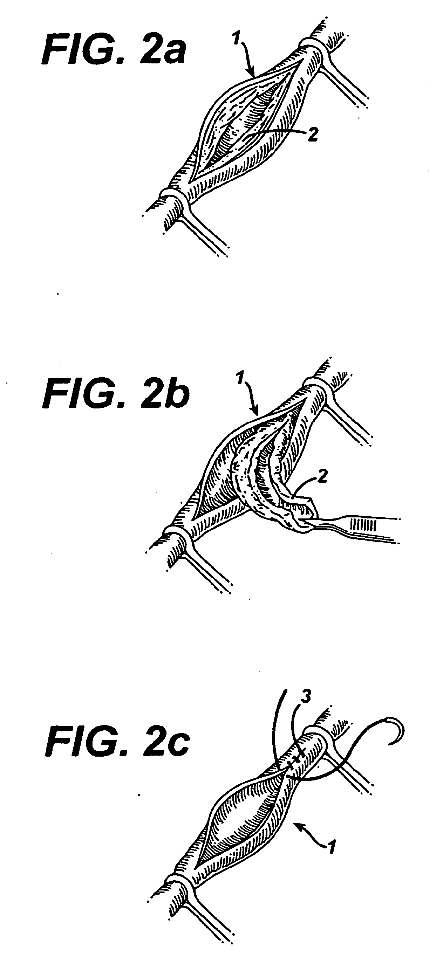

In the past, surgical procedures have been used to remove accumulations of plaque from a blood vessel. For example, as shown in Fig. 1, the carotid artery 1 supplies blood and oxygen to the brain. As shown in Figs. 2a-2c, where the carotid artery 1 has had an accumulation of plaque 2, a carotid endarterectomy has often been performed to surgically open a section of the carotid artery 1 and remove the plaque 2 from the carotid artery. The carotid artery is then sutured closed (Fig. 2c) and blood flow is restored through the carotid artery 1. The carotid endarterectomy has been widely used as a way to reduce the risk of stroke by removing the plaque accumulation from the carotid artery. While often effective, the carotid endarterectomy risks causing the very stroke the procedure is intended to avoid if a cerebral embolism, i.e., a blood clot that breaks loose and travels to the brain, were to occur during the procedure.

Of course, accumulations of plaque can occur almost anywhere in the body. Mechanically compressing the plaque using a balloon angioplasty to widen the lumen within the blood vessel has also been used to treat plaque accumulations in blood vessels as well. Likewise, bypass surgery has been used to graft another vessel through which blood flow may occur to avoid the plaque obstructed blood vessel altogether. Neither of these methods remove the plaque accumulations from the blood vessels however.

Alternatively, drugs such as statins are often ingested or otherwise administered to patients to reduce, or minimize the likelihood of, plaque accumulations in blood vessels. Such drugs, however, may deplete patients of other important substances over time. All of the treatment alternatives practiced to date have drawbacks therefore.

US-6231496 discloses a method of sterilising a female organism by depositing magnetizable metal particles into a uterus of the female organism. Subsequently, a magnet is placed near an external skin surface of the female organism near the uterus to thereby cause at least some of the metal particles to embed into the uterus.

In view of the above, a need exists for systems and methods that more easily and safely treat and remove accumulations of plaque in a blood vessel.

The systems of the invention introduce ferrofluids into the bloodstream of a patient and magnetically manipulate the ferrofluids in order to break up and remove plaque accumulations therewithin. In some embodiments of the systems of the invention, the ferrofluids are introduced locally to a targeted blood vessel using a catheter or micro-catheter. More specifically, a micro-catheter is used to deliver ferrofluids locally to smaller blood vessels, whereas a catheter is used to deliver ferrofluids locally to larger blood vessels. The ferrofluids are then magnetically manipulated to remove the accumulated plaque. In other embodiments, the ferrofluids are intravenously introduced to the bloodstream of the patient. The intravenously introduced ferrofluids are magnetically moved to the site of the accumulated plaque and then further magnetically manipulated to break-up and remove the accumulated plaque. Upon removal of the accumulated plaque, the ferrofluids may be further magnetically manipulated to polish the interior surface of the blood vessel.

In some embodiments, the ferrofluids incorporate abrasive particles that are manipulated along with the ferrofluids to break-up the accumulated plaque in the bloodstream. In other embodiments, the ferrofluids incorporate anti-plaque drugs that are manipulated along with the ferrofluids to break-up the accumulated plaque in the bloodstream. In still other embodiments, the ferrofluids incorporate a combination of abrasive particles and an anti-plaque drug that are manipulated along with the ferrofluids to break-up the accumulated plaque in the bloodstream.

In those embodiments where a micro-catheter is used to introduce the ferrofluids into the bloodstream, the micro-catheter may include at least one sensor at a tip of a guidewire, for example. The at least one sensor helps identify conditions pertaining to the accumulated plaque site and the activity of the ferrofluids at that site in particular. To this end, the at least one sensor may identify conditions such as pressure, temperature, flow, shaft deflection or the like to indicate how the ferrofluids are acting at the accumulated plaque site within the blood vessel. Based on the data sensed by the at least one sensor, the magnetic field, or the position of the patient relative to the magnetic field, may be altered in order to more appropriately manipulate the ferrofluids to break-up and remove the accumulated plaque.

The systems of the invention further provide a magnetic field generator. The magnetic field generator is external of the patient and is used to manipulate or move the ferrofluids within the bloodstream of the patient. The magnetic field generator may induce a vortex, high velocity jets or other motion, in the ferrofluids introduced into the bloodstream of the patient. The magnetically induced vortex, high velocity jets or other motion in the ferrofluids, breaks-up and removes the accumulated plaque in the targeted blood vessel. After the accumulated plaque is removed and the interior surface of the blood vessel polished, the ferrofluids may remain in the patient for eventual consumption by naturally occurring phagocytotic cells, or the ferrofluids may have magnetic components of the ferrofluids magnetically recaptured and removed from the bloodstream using the catheter or micro-catheter, for example.

In some embodiments, the magnetic field generator comprises a tubular member into which the patient is placed. The magnetic field generator in this instance is similar to an MRI or CT scanner system, whereby the patient lies prone on a movable table that is transportable into and out of the tubular member. The tubular member according to this embodiment of the systems and methods of the invention further comprises a movable collar having at least one magnet circumferentially arranged about a portion of the tubular member. The collar having at least one magnet surrounds the body part of the patient having the accumulated plaque. When stationary, the collar having at least one magnet provides a magnetic field sufficient to concentrate intravenously delivered ferrofluids at the intended accumulated plaque site. When rotated, or otherwise moved, the collar having at least one magnet provides a magnetic field sufficient to induce the vortex, high velocity jets or other motion of the ferrofluids at the accumulated plaque site that is used to break-up and remove the accumulated plaque. Of course, independent magnets could instead be used to concentrate the intravenously delivered fluids at the intended accumulated plaque site.

In still other embodiments, the magnetic field generator is a more portable system transportable in emergency vehicles, for example. The portable magnetic field generator comprises a smaller scale tubular member into which the body part having the accumulated plaque is placed. The portable tubular member also comprises a movable collar having at least one magnet that surrounds the accumulated plaque site of the patient when the body part is placed within the portable tubular member. As in the larger scale tubular member, the stationary collar having at least one magnet provides a sufficient magnetic field to concentrate the intravenously delivered ferrofluids at the intended accumulated plaque site, whereas rotation of the collar having the at least one magnet provides a sufficient magnetic field to induce the vortex, high velocity jets or other motion of the ferrofluids that breaks-up and removes the accumulated plaque similar to as described above. The portable tubular member is ideally sufficiently lightweight that it can be managed by a single emergency or other medical professional and placed around the intended body part with minimal movement of the patient.

The systems of the invention thus provide a low profile delivery system for delivering ferrofluids to the bloodstream, whereby the ferrofluids are easily manipulated within a vessel. Rigid mechanical components are not required to be introduced to the bloodstream or to penetrate through the accumulated plaque in a blood vessel. Unintended damage to blood vessels or other organs is minimized as a result.

The systems of the invention simplify the treatment and removal of accumulated plaque from a blood vessel and can require less training for a medical professional administering the ferrofluids to a patient. Where the ferrofluids are introduced intravenously to the bloodstream of a patient, emergency medical personnel or front-line hospitals, rather than specialized stroke and neuro-vascular oriented medical centers, may more easily administer the ferrofluids to a patient. The ready access of such emergency and front-line hospitals equipped with the systems and methods of the invention can minimize the detrimental impact an accumulated plaque can have on a patient. Thus, the systems of the invention provide a safer, simpler, and easier manner of treating and removing accumulated plaque within a blood vessel of a patient.

The above and other features of the invention, including various novel details of construction and combinations of parts, will now be more particularly described with reference to the accompanying drawings and claims. It will be understood that the various exemplary embodiments of the invention described herein are shown by way of illustration only and not as a limitation thereof. The principles and features of this invention may be employed in various alternative embodiments without departing from the scope of the claims.

Embodiments of the invention will now be described by way of example with reference to the accompanying drawings, in which:
Figure 1 illustrates generally a carotid artery with an accumulation of plaque;
Figures 2a-2c illustrate various stages of a carotid endarterectomy practiced in the art;
Figure 3 illustrates a blood vessel with an accumulation of plaque therein;
Figure 4 illustrates a carotid artery with an accumulation of plaque therein;
Figure 5 illustrates lower limb blood vessels in which accumulation of plaque may occur;
Figures 6a-6c illustrate various ferrofluid nanoparticles according to the invention;
Figure 7 illustrates a micro-catheter introducing ferrofluids locally to a blood vessel in a patient;
Figures 8a-8c illustrate various stages of the vortex created by the ferrofluids breaking-up and removing accumulated plaque;
Figure 9 illustrates a magnetic field generator according to one aspect of the invention;
Figure 10 illustrates a patient subjected to the magnetic field generator of Fig. 9 according to the invention;
Figure 11 illustrates a portable magnetic field generator according to another aspect of the invention;
Figure 12 illustrates a patient subject to the portable magnetic field generator of Fig. 11 according to the invention; and
Figure 13 illustrates a micro-catheter guide wire tip having sensors incorporated therewith according to another aspect of the invention.

Fig. 3 illustrates an exemplary blood vessel 10 of the vasculature of a human anatomy. The blood vessel 10 is shown as having an accumulation of plaque 20 extending along an interior surface of the blood vessel. The accumulated plaque 20 narrows the blood-flow passageway through the blood vessel 10 increasing the risk of hypoxia, stroke or other undesirable maladies and conditions in a patient. The accumulation of plaque 20 may occur anywhere in the vasculature of the human anatomy, but is often formed in the arterial blood vessels of the neck, heart and the lower limbs of a patient.

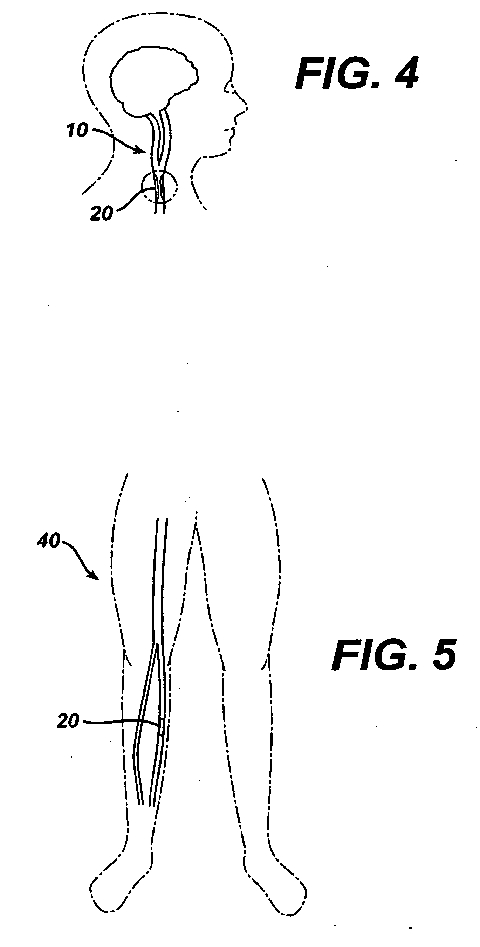

Fig. 4 illustrates accumulations of plaque 20 located in the carotid artery 10, for example. Fig. 5 illustrates accumulations of plaque 20 located in blood vessels of the leg 40, for example. The systems for treating accumulations of plaque according to the invention may be applied to treat accumulated plaque located anywhere in the vasculature of the human anatomy. The artisan should readily appreciate therefore that the accumulations of plaque shown and described herein with respect to either of the carotid artery and blood vessels of the lower limbs are for illustrative purposes only. Similar systems and methods may be used to treat accumulations of plaque at other locations in the vasculature of a patient as well using the systems of the invention.

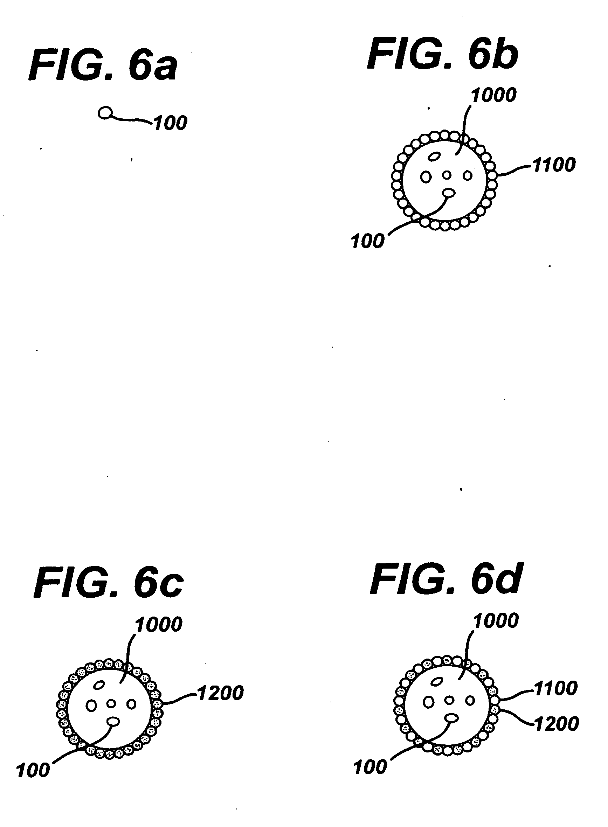

Figs. 6a-6c illustrate various configurations of a ferrofluid according to the invention. The ferrofluids may be colloids with magnetic particles of nanometer or micron size dispersed in a carrier fluid. The carrier fluid may be water, water-alcohol mixtures, or a variety of hydrocarbons, such as paraffin oil or synthetic esters, for example. The magnetic particles may be comprised of materials such as magnetite or cobalt ferrite, for example. The artisan should appreciate that other materials having similar properties as known in the art are also readily usable as the carrier fluids or magnetic particles, as appropriate, with the systems of the invention.

Fig. 6a more specifically illustrates a single magnetic particle 100 according to the invention. A plurality of magnetic particles 100 in colloid solution within a carrier fluid comprises the ferrofluids that are delivered to the bloodstream of a patient to break-up and remove accumulated plaque according to the invention. Each single magnetic particle 100 is preferably 5-500 nanometers in size and is comprised of bio-compatible material of sufficient radiopacity as to be visible using known fluoroscopy techniques, for example. However, non-radiopaque magnetic particles may also be used if the ferrofluid in the vasculature is visualized by means other than fluoroscopy. Of course, the artisan will appreciate that the ferrofluids may also comprise a combination of different magnetic particles 100 dispersed within the colloid.

Fig. 6b illustrates a ferrofluid droplet 1000 having abrasive particles 1100 added thereto. The abrasive particles 1100 are thus added to the ferrofluid colloid. The abrasive particles 1100 will be displaced to a surface of the ferrofluid droplet 1000 (as shown in Fig. 6b) once the ferrofluid droplet 1000 is exposed to a sufficient magnetic field. Other abrasive particles may also be expelled to the surface of the ferrofluid droplets because of surface characteristics. Such surface characteristics may include hydrophobic functional groups that would expel the abrasive particle to the surface of a ferrofluid droplet having a hydrophilic carrier fluid. Alternately, abrasive particles having hydrophilic functional groups on their surface would be expelled to the surface of a ferrofluid having a hydrophobic carrier fluid. The abrasive particles may be comprised of a bio-compatible abrasive material, such as beads made of glass, polymers, magnetic or non-magnetic metals, or any other bio-compatible abrasive material known in the art that is able to mix with the ferrofluid droplet 1000.

Fig. 6c illustrates a ferrofluid droplet 1000 having drug carrier particles 1200 dispersed therein and being impregnated with an anti-plaque drug, some of which may be expelled to the surface of the ferrofluid droplet 1000. The anti-plaque drug may comprise a plaque reducing or plaque formation inhibiting drug as known or later developed in the art. The anti-plaque drug may be bound to the surface of the magnetic particles 100 dispersed within the ferrofluids. Alternatively, the anti-plaque drug may be bonded to the abrasive particles 1100, may be carried in pores or internal cavities of the magnetic particles 100, or may be added to the colloid of ferrofluids via carrier particles 1200 (Fig. 6d) designed to carry the anti-plaque drug within the ferrofluids.

Fig. 6d illustrates a ferrofluid droplet 1000 having carrier particles 1200 that carry an anti-plaque drug. As shown in Fig. 6d, the ferrofluid droplet 1000 also contains abrasive particles 1100, which have been displaced to the surface of the ferrofluid droplet 1000 after exposure to a magnetic field according to the systems of the invention.

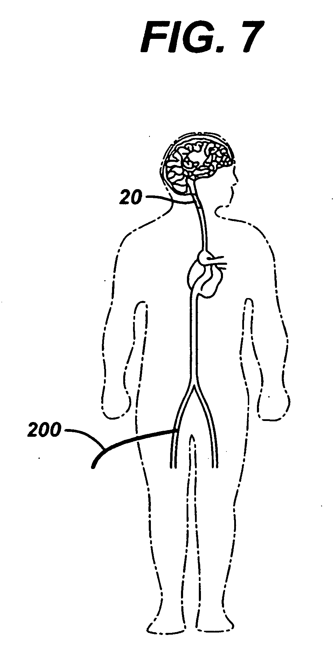

In practice, ferrofluids are delivered to the site of the accumulated plaque in conventional manner using a catheter, a micro-catheter or intravenously. As shown in Fig. 7, wherein a micro-catheter 200 is used to deliver ferrofluids to the carotid artery, for example, the ferrofluids are generally delivered directly to the site of the accumulated plaque 20. On the other hand, when intravenous delivery of the ferrofluids is used (not shown), the ferrofluids are delivered systemically to the bloodstream of the patient and then subsequently magnetically manipulated to the site of the accumulated plaque.

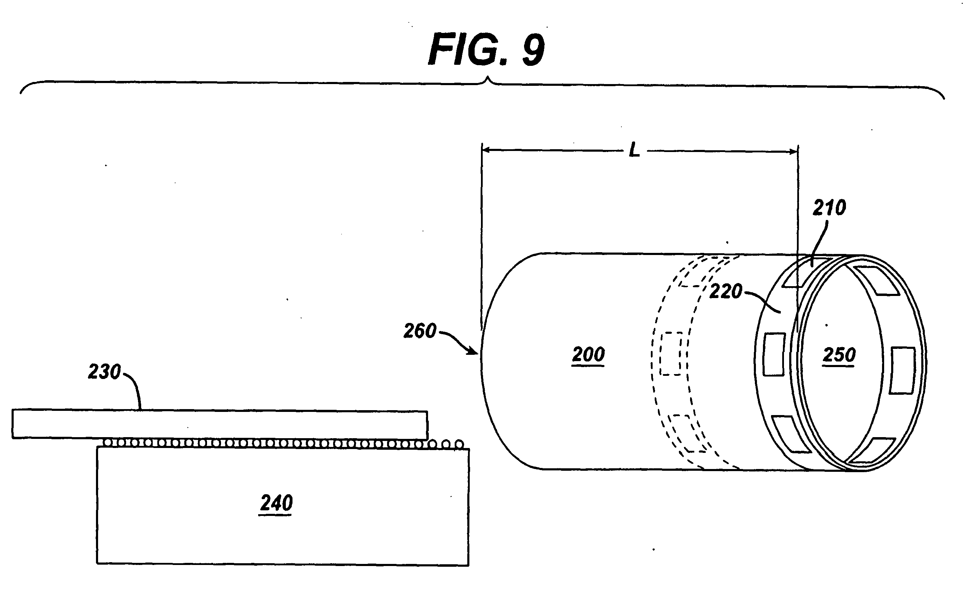

Once the ferrofluids are located at the site of the accumulated plaque, whether by direct catheter or micro-catheter delivery or by indirect intravenous delivery with subsequent magnetic manipulation, the ferrofluids are then subjected to a magnetic field generated by an external magnetic field generator, as will be discussed in more detail below with respect to Figs 9-12. The external magnetic field generator externally encircles a body part of the patient whereat the accumulated plaque is located. By moving or rotating the magnetic field, the ferrofluids within the blood vessel of the encircled body part are also rotated or moved.

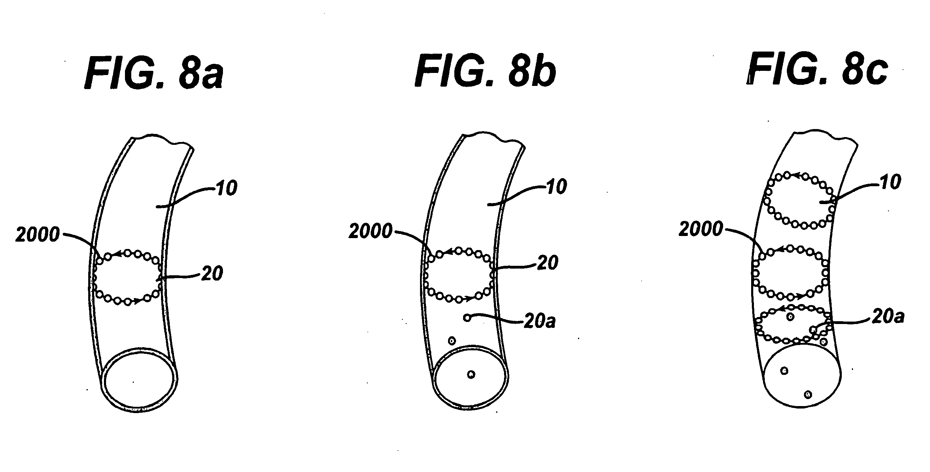

Figs. 8a-8c illustrate various stages of rotating the ferrofluids at the site of the accumulated plaque 20 within a blood vessel 10. For example, Fig. 8a illustrates the ferrofluids being rotated in the blood vessel 10. By rotating the external magnetic field sufficiently the ferrofluids are induced to form a vortex 2000 within the blood vessel 10. Ideally, as shown in Figs. 8a-8c, the vortexed ferrofluids 2000 rotate along the inner surface of the blood vessel 10 to break-up and remove the accumulated plaque 20 from the blood vessel 10. The ferrofluids may instead be induced by the magnetic field to produce high velocity jets or other motion in the ferrofluids to break-up and remove the accumulated plaque, although the inducement and use of the vortex 2000 is described in greater detail herein.

In Fig. 8a, for example, the vortex 2000 of ferrofluids has just begun and the accumulated plaque 20 is largely intact along the interior surface of the blood vessel 10. In Fig. 8b, the vortex of ferrofluids 100 has taken place for awhile and has broken-up or otherwise detached portions of the accumulated plaque 20 from the blood vessel 10. Remnants 20a of the plaque 20 broken-up by the vortexed rotation of the ferrofluids 100 are evident in Fig. 8b, for example. The swath or width of plaque broken-up by the vortexed ferrofluids is generally that portion of accumulated plaque 20 contacted directly by the ferrofluids 100. The wider the band of ferrofluids, the more accumulated plaque is contacted and removed at one time. The ferrofluids may be magnetically manipulated to contact various portions of the accumulated plaque along the interior surface of the blood vessel 10 until the entire area of accumulated plaque has been contacted and removed by the ferrofluids. Ideally, the remnants 20a that occur as a result of the vortexed ferrofluids action on the accumulated plaque 20 are generally dispersed through the blood vessel 10 without event.

In Fig. 8c the ferrofluids 100 have been manipulated to various locations along the blood vessel and the accumulated plaque 20 has been removed therefrom by the vortexed ferrofluids 10, leaving only the loosened remnants 20a of the accumulated plaque for dispersal through the bloodstream. Although the rotation of the vortex 2000 shown in Figs. 8a-8c is counter-clockwise, the artisan will appreciate that clockwise, or other, rotational directions are also usable according to the systems and method of the invention.

Where the ferrofluids 1000 used have magnetic particles 100 but are devoid of abrasives 1100 or anti-plaque drugs, the vortexed ferrofluids 2000 may simply detach the accumulated plaque 20 from the blood vessel 10, rather than fully breaking up the accumulated plaque. Thereafter, the detached plaque 20 may be mechanically extracted from the blood vessel using a catheter or micro-catheter in conventional manner. The risks of the additional extraction procedure are self-evident, but such a drug-less procedure minimizes drug-induced side-effects that can occur when anti-plaque drugs are used to treat an accumulation of plaque.

Where the ferrofluids 1000 have magnetic particles 100 and are impregnated with abrasive particles 1100, the abrasive particles 1100 work in combination with the vortex 2000 to break-up and remove the accumulated plaque 20, ideally without need for mechanically extracting any part of the plaque. The absence of the additional extraction procedure minimizes risk of puncture or other damage to the blood vessel in which the accumulated plaque is located. The absence of an anti-plaque drug minimizes or eliminates the risk of side-effects associated with the use of such drugs.

Where the ferrofluid droplets 1000 with magnetic particles 100 are impregnated with an anti-plaque drug via carrier particles 1200, the anti-plaque drug work in combination with the vortex 2000 to break-up and remove the accumulated plaque 20, ideally without need to mechanically extract any portion of the thrombus. Though the risk of side-effects is present due to the use of the anti-plaque drug in this instance, the risks attendant with the additional extraction procedure of a drugless procedure are minimized.

Of course, the artisan will appreciate that where a combination of abrasive particles 1100 and an anti-plaque drug is used with the ferrofluid droplets 1000, the combination works with the vortex 2000 to break-up and remove the accumulated plaque 20, ideally also without the need for mechanical extraction of the plaque. Thus, the use of such a combination would minimize at least the risks associated with the additional extraction procedure.

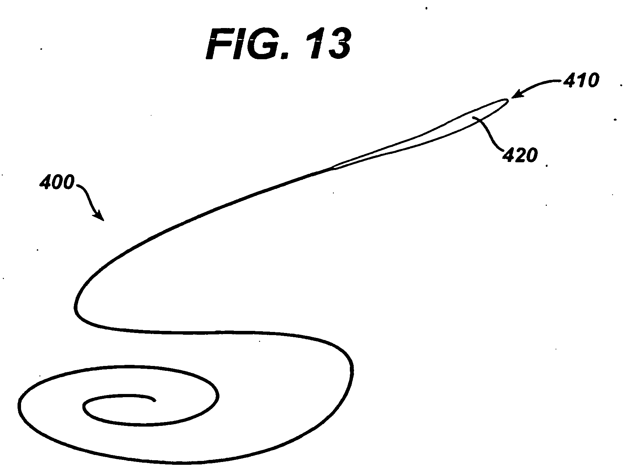

Once the accumulated plaque 20 is removed, the components of the ferrofluids that are magnetic, i.e., the magnetic particles 100, the abrasive particles 1100 if made of magnetic materials, and any carrier particles 1200 if made of magnetic materials, may either remain in the patient's bloodstream, or may be magnetically recaptured and removed from the body using a catheter or micro-catheter by directing the magnetic components out of the bloodstream through the catheter or micro-catheter, as the case may be. The catheter or micro-catheter in this instance could include a guide-wire having a magnetic tip (Fig. 13), for example, that would guide the magnetic components of the ferrofluids through and out of the blood vessel 10, via the catheter or micro-catheter, as the guide-wire is extracted from the blood vessel in conventional manner.

Referring to Fig. 9, a magnetic field generator system is shown. The magnetic field generator system comprises a tubular member 200, at least one magnet 210, a collar 220, a movable table 230 and a base 240. The at least one magnet 210 is placed in the collar 220 positioned along a circumference of the tubular member 200. The collar 220 is movable or rotatable. Of course, the artisan should readily appreciate that the at least one magnet 210, shown as a plurality of magnets 210 in Fig. 9, could as well be comprised of a single magnetic band within the collar 220. Moreover, where provided, the plurality of magnets may be arranged on or within the collar 220 in a variety of configurations.

As shown in Fig. 9, the collar 220 is positioned near a first end 250 of the tubular member. The collar 220 is translatable along the length L of the tubular member 200 such that the collar 220 can be positioned anywhere along the length L of the tubular member 200 between the first end 250 and a second end 260. For example, the collar 220 is shown in dashed lines about midway between the first end 250 and the second end 260 in Fig. 9 as well. Regardless of the translational position of the collar 220 along the tubular member 200, the collar 220 is movable or rotatable about the circumference of the tubular member. In this manner, the magnetic field generated by the at least one magnet 210 in the collar 220 is applied to the patient received within the tubular member 200. Furthermore, the collar 220 may be tilted, allowing the collar to rotate about any axis within three-dimensional space.

Referring still to Fig. 9, the movable table 230 is movably mounted to a base 240. The table 230, for example, may move into and out of the tubular member 200 in order to position a patient in the tubular member 200 for appropriate orientation relative to the at least one magnet 210 and collar 220.

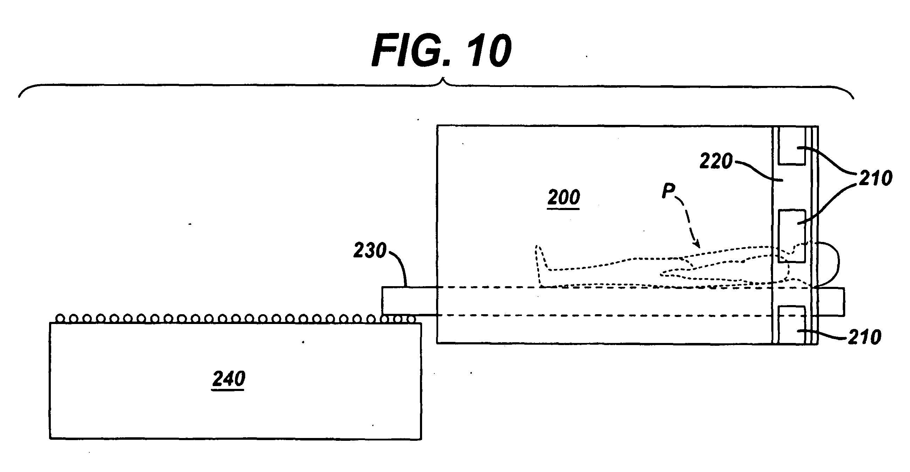

Fig. 10 illustrates more specifically a patient P positioned on the table 230 within the tubular member 200. The neck of the patient P, for example, is oriented generally in line with the at least one magnet 210 in the collar 220. Such an orientation of the patient's neck in this manner is contemplated when an accumulation of plaque in a carotid artery of a patient is sought to be treated using the systems of the invention. In practice, the collar 220 is moved or rotated to generate a magnetic field about the neck of the patient P. The magnetic field induces the vortex 2000, high velocity jets or other motion, in the ferrofluids previously delivered to the patient. The accumulated plaque is then broken-up and removed by the vortexed ferrofluids as described above according to the type of ferrofluid droplets 1000 used. Rotation or movement of the collar 220 and the at least one magnet 210 is stopped, and the patient is removed from the tubular member after the accumulated plaque has been broken-up and removed.

A conventional switch (not shown) may be used with the magnetic field generator in order to move or rotate the collar 220 when desired. Likewise, a conventional switch and moving means, such as a belt drive, rollers, glide systems or combinations thereof, may be used to move the table 230 along the base 240 and into and out of the tubular member 200 when desired. The magnetic field generator is otherwise powered by conventional means.

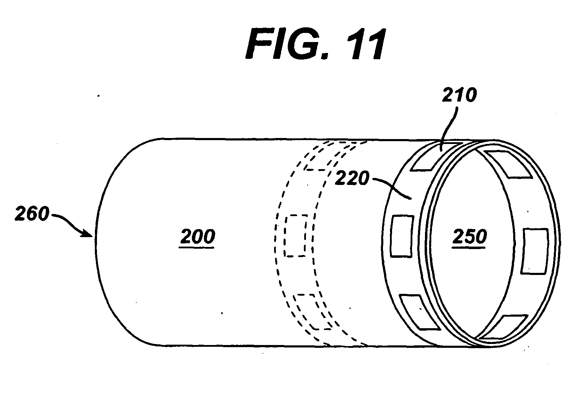

Fig. 11 illustrates a smaller scale portable magnetic field generator system according to the systems of the invention, wherein like reference numbers are used to identify like features. As shown in Fig. 11, the magnetic field generator comprises a tubular member 200, at least one magnet 210, and a collar 220. The tubular member 200 has a first end 250 and a second end 260. The at least one magnet 210 is placed in the collar 220 positioned along a circumference of the tubular member 200. Of course, the artisan should readily appreciate that, as with the magnetic field generator of Fig. 9, the at least one magnet 210, shown as a plurality of magnets 210 in Fig. 11, could as well be comprised of a single magnetic band within the collar 2200. The collar 220 is similarly movable or rotatable as in earlier described embodiments.

The magnetic field generator of Fig. 11 and operation thereof is generally the same as that described with reference to the magnetic field generator of Fig. 9 except that the tubular member 200 of Fig. 11 receives only a body part of a patient rather than the entire patient P (Fig. 10). Thus, the dimensions of the tubular member 200 and associated components of Fig. 11 are proportionately diminished from those of the tubular member 200 and associated components of Fig. 9. The artisan should readily appreciate the dimensional differences, which are omitted herein for brevity.

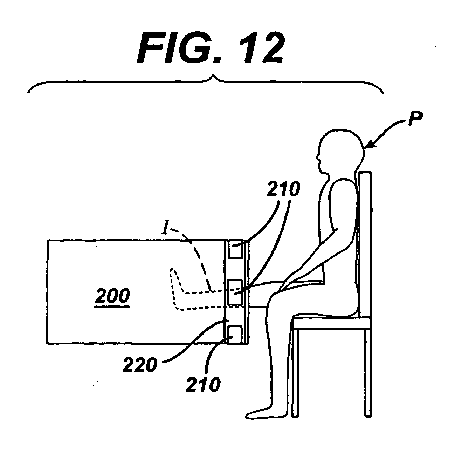

Fig. 12 illustrates a lower limb 1 of a patient P being received within the tubular member 200 of the magnetic field generator of Fig. 11. Such lower limb 1 would be received within the tubular member 200 when an accumulation of plaque exists in the lower limb. The collar 220 and the at least one magnet 210 are then rotated to induce the vortex 2000 in the ferrofluids within the blood vessel whereat the accumulated plaque is located. The accumulated plaque is thus broken-up and removed in a manner consistent with the type of ferrofluid droplets 1000 used. The magnetic field is then terminated. After break-up and removal of the accumulated plaque, the ferrofluids either remain in the bloodstream for eventual consumption by naturally occurring macrophages, or the magnetic components of the ferrofluids are removed from the bloodstream using a magnetic tipped guide-wire and micro-catheter in conventional manner as discussed above. Rotation or movement of the collar 220 and the at least one magnet 210 is stopped, and the patient's body part is removed from the tubular member 200 after the plaque has been broken-up and removed.

Ideally, the portable magnetic field generator is transportable using a transport device, such as a conventional dolly-like apparatus, for example, similar to the manner in which oxygen tanks are commonly transported. Preferably, the transport device would include a power supply system to which the magnetic field generator could be connected. Of course, the artisan should readily appreciate that, where provided, the power supply system would provide sufficient power to generate a magnetic field of sufficient gradient to induce the vortex of the ferrofluids used to break-up and remove the accumulated plaque. Alternatively, the portable magnetic field generator could be powered by other conventional non-portable means.

Fig. 13 illustrates a guide-wire 400 for use with a conventional catheter or micro-catheter according to the systems of the invention. The guide-wire 400 comprises a magnetic tip 410 and at least one sensor 420 at its tip, but is otherwise conventional. The at least one sensor 420 may be inserted through the catheter or micro-catheter with the ferrofluids 100 to the site of the accumulated plaque. The at least one sensor 420 may be used to measure pressure, temperature, guide-wire tip deflection, or other conditions occurring at the site of the accumulated plaque in order to indicate how the ferrofluids are performing. For example, where the at least one sensor 420 is a tip deflection sensor, the sensor 420 will detect the amount or angle of deflection of the guide-wire tip 410 within the blood vessel, wherein the bending or deflection of the guide-wire tip complies with the direction of movement or rotation of the magnetic field generated by the magnetic field generator of Fig. 9 or Fig. 11. If the amount or degree of angular deflection of the tip 410 is insufficient, one can presume that the intended vortex 2000 of the ferrofluids is not being created by the generated magnetic field. The strength, geometry, or gradient of the magnetic field can thus be altered, or the position of the patient can be altered to more appropriately align the site of the accumulated plaque with the magnets, and hence the magnetic field, of the magnetic field generator. Where provided, the magnetic tip 410 of the guide-wire 400 is also usable to magnetically attract and remove the magnetic components of the ferrofluids from the bloodstream of the patient after the accumulated plaque has been removed, as discussed earlier. Still further, the at least one sensor may be provided on a tip of the catheter or micro-catheter. Alternatively, the at least one sensor can simply be omitted.

## Claims

1. A system for removing accumulated plaque (2) in a blood vessel (1) of a patient, the system comprising:
a magnetically manipulable ferrofluid comprising magnetic particles (100) dispersed in a carrier fluid suitable to be disposed within the blood vessel (1);
a magnetic field generator, arranged to provide a magnetic field sufficient and adapted to induce a vortex (2000), high-velocity jet or other motion of the ferrofluids at the accumulated plaque site suitable to remove the accumulated plaque;
a catheter or a micro-catheter for delivering the ferrofluids directly to a site of the accumulated plaque (2) within the bloodstream; and
at least one sensor (420) on or near a tip of the catheter or micro-catheter for determining the activity of the ferrofluids at the site of the accumulated plaque.

2. The system of claim 1, wherein the carrier fluid is one of a water-based, a water-alcohol-based, or a hydrocarbon-based carrier fluid.

3. The system of claim 2, wherein the carrier fluid is hydrophilic.

4. The system of claim 2, wherein the carrier fluid is hydrophobic.

5. The system of claim 2, wherein the ferrofluids further comprise abrasive particles (1100) mixed into the ferrofluids, the abrasive particles (1100) displaceable to a surface of the ferrofluids when exposed to a magnetic field.

6. The system of claim 3, wherein the ferrofluids further comprise hydrophobic abrasive particles (1100) mixed into the ferrofluids, the hydrophobic abrasive particles (1100) displaceable to a surface of the ferrofluids when subject to the hydrophilic carrier fluid,

7. The system of claim 4, wherein the ferrofluids further comprise hydrophilic abrasive particles (1100) mixed into the ferrofluids, the hydrophilic abrasive particles (1100) displaceable to a surface of the ferrofluids when subject to the hydrophobic carrier fluid.

8. The system of claim 2, wherein the ferrofluids further comprise an anti-plaque drug.

9. The system of claim 8, wherein the anti-plaque drug is bonded to one of the magnetic particles (100) or the abrasive particles (1100), is mixed into the carrier fluids, or is added to the ferrofluids by carrier particles (1200) to deliver the anti-plaque drug throughout the ferrofluids.

10. The system of claim 1, wherein the ferrofluids further comprise a combination of abrasive particles (1100) and an anti-plaque drug carried with the ferrofluids.

11. The system of claim 1, further comprising:
a magnetically tipped guide-wire (400).

12. The system of claim 11, further comprising;
at least one sensor (420) on the tip of one of the catheter, the micro-catheter, or the guide-wire (400) for determining the activity of the ferrofluids at the site of the accumulated plaque (2).

13. The system of claim 12, wherein one of the at least one sensor (420) is a deflection sensor to determine the amount of deflection of the guide-wire tip (410) as an indication of the activity of the ferrofluids at the site of the occluding thrombus (2).

14. The system of claim 13, wherein the at least one sensor (420) further comprises a temperature or pressure sensor.

15. The system of claim 14, wherein a strength, geometry or gradient of the magnetic field is determined based on the data sensed from the at least one sensor (420).

16. The system of claim 1, wherein the magnetic field generator further comprises:
a tubular member (200) having a first end (250) and a second end (260);
a collar (220) positionable along an exterior circumference of the tubular member (200) between the first end (250) and the second end (260) of the tubular member (200);
at least one magnet (210) provided with the collar (220);
a movable table (230) movably mounted to a base (240), the movable table (230) oriented to hold the patient and movable into and out of the tubular member (200) with the patient aboard said table (230); and
means for powering the movable table (230) and the collar (220).

17. The system of claim 1, wherein the magnetic field generator further comprise:
a tubular member (200) having a first end (250) and a second end (260);
a collar (220) positionable along an exterior circumference of the tubular member (200) between the first end (250) and the second end (260) of the tubular member (200);
at least one magnet (210) provided with the collar (220); and
means for powering the collar (220), wherein only a body part of the patient is received within the tubular member (200).

18. The system of claim 16 or 17, wherein the collar (220) is at least one of rotatable or movable.

19. The system of claim 16 or 17, wherein the at least one magnet (210) is a permanent magnet.

20. The system of claim 16 or 17, wherein the at least one magnet (210) is an electromagnet.

21. The system of claim 1, further comprising an intravenous delivery of the ferrofluids to the bloodstream.

22. The system of claim 21, wherein the ferrofluids are concentrated to a site of the accumulated plaque (2) prior to the removal of the accumulated plaque (2) by the ferrofluids.

23. The system of claim 1, wherein the ferrofluids are arranged to break up and remove the accumulated plaque (2) and polish an interior surface of the blood vessel.

24. The system of claim 11, wherein the guide-wire (400) with the magnetic tip (410) further comprises a magnetic re-capture device for re-capturing and directing magnetic components (100) within the ferrofluids through the catheter or micro-catheter to remove the magnetic components (100) of the ferrofluids from the blood vessel (1).

## Patentansprüche

1. System zum Beseitigen angesammelter Plaque (2) in einem Blutgefäß (1) eines Patienten, wobei das System aufweist:
ein magnetisch beeinflussbares Ferrofluid, das magnetische Teilchen (100) aufweist, welche in einem Trägerfluid dispergiert sind, das geeignet ist, in das Blutgefäß (1) gebracht zu werden;
einen Generator für ein Magnetfeld, der so angeordnet ist, dass er ein Magnetfeld zur Verfügung stellt, welches ausreichend und dazu ausgelegt ist, eine wirbelnde Bewegung (200), einen Hochgeschwindigkeitsstrahl oder eine andere Bewegung der Ferrofluide an dem Ort der angesammelten Plaque hervorzurufen, die geeignet ist, die angesammelte Plaque zu beseitigen;
einen Katheter oder einen Mikrokatheter zum Zuführen der Ferrofluide direkt an einen Ort der angesammelten Plaque (2) innerhalb des Blutstroms;
wenigstens einen Sensor (420) auf oder nahe einer Spitze des Katheters oder Mikrokatheters zum Bestimmen der Aktivität der Ferrofluide an dem Ort der angesammelten Plaque.

2. System nach Anspruch 1, bei dem das Trägerfluid eines aus einem auf Wasser basierenden, einem auf Wasser-Alkohol basierenden oder einem auf Kohlenwasserstoff basierenden Trägerfluid ist.

3. System nach Anspruch 2, bei dem das Trägerfluid hydrophil ist.

4. System nach Anspruch 2, bei dem das Trägerfluid hydrophob ist.

5. System nach Anspruch 2, bei dem die Ferrofluide weiter Schleifpartikel (1100), die in die Ferrofluide eingemischt sind, aufweisen, wobei die Schleifpartikel (1100) an eine Oberfläche der Ferrofluide verlagerbar sind, wenn sie einem Magnetfeld ausgesetzt werden.

6. System nach Anspruch 3, bei dem die Ferrofluide weiter hydrophobe Schleifpartikel (1100), die in die Ferrofluide eingemischt sind, aufweisen, wobei die hydrophoben Schleifpartikel (1100) an eine Oberfläche der Ferrofluide verlagerbar sind, wenn sie unter dem Einfluss des hydrophilen Trägerfluids sind.

7. System nach Anspruch 4, bei dem die Ferrofluide weiter hydrophile Schleifpartikel (1100), die in die Ferrofluide eingemischt sind, aufweisen, wobei die hydrophilen Schleifpartikel (1100) an eine Oberfläche der Ferrofluide verlagerbar sind, wenn sie unter dem Einfluss des hydrophoben Trägerfluids sind.

8. System nach Anspruch 2, bei dem die Ferrofluide weiter eine gegen Plaque wirkende Arznei aufweisen.

9. System nach Anspruch 8, bei dem die gegen Plaque wirkende Arznei entweder an die magnetischen Teilchen (100) oder die Schleifpartikel (1100) gebunden ist, in die Trägerfluide gemischt wird oder zu den Ferrofluiden mittels Trägerteilchen (1200) hinzugefügt wird, um die gegen Plaque wirkende Arznei durch die Ferrofluide zuzuführen.

10. System nach Anspruch 1, bei dem die Ferrofluide weiter eine Kombination aus Schleifpartikeln (1100) und einer gegen Plaque wirkenden Arznei aufweisen, die mit den Ferrofluiden transportiert wird.

11. System nach Anspruch 1, das weiter aufweist:
einen mit einer magnetischen Spitze versehenen Führungsdraht (400).

12. System nach Anspruch 11, das weiter aufweist:
wenigstens einen Sensor (420) auf der Spitze entweder des Katheters, des Mikrokatheters oder des Führungsdrahtes (400) zum Bestimmen der Aktivität der Fluide an dem Ort der angesammelten Plaque (2).

13. System nach Anspruch 12, bei dem einer aus dem wenigstens einen Sensor (420) ein Ablenkungssensor ist, um den Betrag der Ablenkung der Spitze (410) des Führungsdrahtes als eine Angabe der Aktivität der Ferrofluide an dem Ort des verstopfenden Thrombus (2) zu bestimmen.

14. System nach Anspruch 13, bei dem der wenigstens eine Sensor (420) weiter einen Temperatur- oder Drucksensor aufweist.

15. System nach Anspruch 14, bei dem eine Stärke, eine Geometrie oder ein Gradient des Magnetfeldes basierend auf den Daten, die von dem wenigstens einen Sensor (420) abgefühlt werden, bestimmt wird.

16. System nach Anspruch 1, bei dem der Generator für ein Magnetfeld weiter aufweist:
ein rohrförmiges Element (200) mit einem ersten Ende (250) und einem zweiten Ende (260);
einen Kragen (220), der entlang einem Außenumfang des rohrförmigen Elementes (200) zwischen dem ersten Ende (250) und dem zweiten Ende (260) des rohrförmigen Elementes (200) positionierbar ist;
einen Magneten (210), der mit dem Kragen (220) bereitgestellt wird;
einen bewegbaren Tisch (230), der bewegbar an einer Basis (240) angebracht ist, wobei der bewegbare Tisch (230) derart ausgerichtet ist, dass er den Patienten hält, und in das rohrförmige Element (200) hinein und aus diesem heraus mit dem Patienten auf dem Tisch (230) bewegbar ist; und
eine Einrichtung zum Versorgen des bewegbaren Tisches (230) und des Kragens (220) mit Energie.

17. System nach Anspruch 1, bei dem der Generator für ein Magnetfeld weiter aufweist:
ein rohrförmiges Element (200) mit einem ersten Ende (250) und einem zweiten Ende (260);
einen Kragen (220), der entlang einem Außenumfang des rohrförmigen Elementes (200) zwischen dem ersten Ende (250) und dem zweiten Ende (260) des rohrförmigen Elementes (200) positionierbar ist;
wenigstens einen Magneten (210), der mit dem Kragen (220) bereitgestellt wird; und
eine Einrichtung zum Versorgen des Kragens (220) mit Energie, wobei nur ein Körperteil des Patienten innerhalb des rohrförmigen Elementes (200) aufgenommen ist.

18. System nach Anspruch 16 oder 17, bei dem der Kragen (220) wenigstens drehbar oder bewegbar ist.

19. System nach Anspruch 16 oder 17, bei dem der wenigstens eine Magnet (210) ein Dauermagnet ist.

20. System nach Anspruch 16 oder 17, bei dem der wenigstens eine Magnet (210) ein Elektromagnet ist.

21. System nach Anspruch 1, weiter mit einer intravenösen Zuführung der Ferrofluide in den Blutstrom.

22. System nach Anspruch 21, bei dem die Ferrofluide vor dem Entfernen der angesammelten Plaque (2) durch die Ferrofluide auf einen Ort der angesammelten Plaque (2) konzentriert werden.

23. System nach Anspruch 1, bei dem die Ferrofluide so eingerichtet sind, dass sie die angesammelte Plaque (2) aufbrechen und entfernen und eine Innenfläche des Blutgefäßen polieren.

24. System nach Anspruch 11, bei dem der Führungsdraht (400) mit der magnetischen Spitze (410) weiter ein magnetisches Wiedereinfangelement zum Wiedereinfangen und Leiten magnetischer Komponenten (100) innerhalb der Ferrofluide durch den Katheter oder Mikrokatheter, um die magnetischen Komponenten (100) der Ferrofluide aus dem Blutgefäß (1) zu entfernen.

## Revendications

1. Système destiné à éliminer l'accumulation de plaque (2) dans un vaisseau sanguin (1) d'un patient, le système comprenant :
■ un ferrofluide manipulable magnétiquement comprenant des particules magnétiques (100) dispersée dans un fluide support adapté pour être disposé dans le vaisseau sanguin (1) ;
■ un générateur de champ magnétique, disposé de façon à fournir un champ magnétique suffisant et adapté pour induire un vortex (2000), un jet à grande vitesse ou un autre mouvement des ferrofluides sur le site de plaque accumulée adapté pour éliminer la plaque accumulée ;
■ un cathéter ou un micro-cathéter pour distribuer les ferrofluides directement sur un site de l'accumulation de plaque (2) dans le flux sanguin ; et
■ au moins un capteur (420) sur ou près d'une pointe du cathéter ou micro-cathéter afin de déterminer l'activité des ferrofluides sur le site de l'accumulation de plaque.

2. Système selon la revendication 1, dans lequel le fluide support est un fluide support à base d'eau, à base d'eau et alcool, ou à base d'hydrocarbure.

3. Système selon la revendication 2, dans lequel le fluide support est hydrophile.

4. Système selon la revendication 2, dans lequel le fluide support est hydrophobe.

5. Système selon la revendication 2, dans lequel les ferrofluides comprennent en outre des particules abrasives (1100) mélangées dans les ferrofluides, lesdites particules abrasives (1100) pouvant être déplacées vers une surface des ferrofluides lorsqu'elles sont exposées à un champ magnétique.

6. Système selon la revendication 3, dans lequel les ferrofluides comprennent en outre des particules abrasives hydrophobes (1100) mélangées dans les ferrofluides, lesdites particules abrasives hydrophobes (1100) pouvant être déplacées vers une surface des ferrofluides lorsqu'elles sont soumises à un fluide support hydrophile.

7. Système selon la revendication 4, dans lequel les ferrofluides comprennent en outre des particules abrasives hydrophiles (1100) mélangées dans les ferrofluides, les particules abrasives hydrophiles (1100) pouvant être déplacées vers une surface des ferrofluides quand elles sont soumises à un fluide support hydrophobe.

8. Système selon la revendication 2, dans lequel les ferrofluides comprennent en outre un médicament anti-plaque.

9. Système selon la revendication 8, dans lequel le médicament anti-plaque est lié à une des particules magnétiques (100) ou des particules abrasives (1100) est mélangé dans les fluides supports, ou est ajouté aux ferrofluides par des particules supports (1200) pour fournir le médicament anti-plaque à travers les ferrofluides.

10. Système selon la revendication 1, dans lequel les ferrofluides comprennent en outre une combinaison de particules abrasives (1100) et un médicament anti-plaque porté avec les ferrofluides.

11. Système selon la revendication 1, comprenant en outre : un fil-guide à pointe magnétique (400).

12. Système selon la revendication 11, comprenant en outre
au moins un capteur (420) sur la pointe d'un cathéter, micro-cathéter, ou fil-guide (400) pour déterminer l'activité des ferrofluides sur le site de la plaque accumulée (2).

13. Système selon la revendication 12, dans lequel un d'au moins un capteur (420) est un capteur à déflexion pour déterminer l'ampleur de la déflexion de la pointe de fil-guide (410) comme indication de l'activité des ferrofluides sur le site du thrombus occlusif (2).

14. Système selon la revendication 13, dans lequel ledit capteur (420) comprend en outre un capteur de température ou de pression.

15. Système selon la revendication 14, dans lequel une résistance, la géométrie ou un gradient du champ magnétique est déterminé sur la base des données détectées depuis ledit capteur (420).

16. Système selon la revendication 1, dans lequel le générateur de champ magnétique comprend en outre :
■ un élément tubulaire (200) ayant une première extrémité (250) et une seconde extrémité (260) _{;}
■ un collier (220) pouvant être positionné le long d'une circonférence extérieure de l'élément tubulaire (200) entre la première extrémité (250) et la seconde extrémité (260) de l'élément tubulaire (200) _{;}
■ au moins un aimant (210) doté du collier (220) ;
■ une table mobile (230) montée de manière mobile sur une base (240), la table mobile (230) étant orientée de façon à tenir le patient et mobile dans et hors de l'élément tubulaire (200) avec le patient placé sur ladite table (230) ; et
■ des moyens pour alimenter la table mobile (230) et le collier (220).

17. Système selon la revendication 1, dans lequel le générateur de champ magnétique comprend en outre :
■ un élément tubulaire (200) ayant une première extrémité (250) et une seconde extrémité (260) _{;}
■ un collier (220) pouvant être positionné le long d'une circonférence extérieure de l'élément tubulaire (200) entre la première extrémité (250) et la seconde extrémité (260) de l'élément tubulaire (200) _{;}
■ au moins un aimant (210) fourni avec le collier (220) et
■ des moyens pour alimenter le collier (220) dans lesquels seule une partie du corps du patient est reçue dans l'élément tubulaire (200).

18. Système selon la revendication 16 ou 17, dans lequel le collier (220) est au moins rotatif ou mobile.

19. Système selon la revendication 16 ou 17, dans lequel ledit aimant (210) est un aimant permanent.

20. Système selon la revendication 16 ou 17, dans lequel ledit aimant (210) est un électro-aimant.

21. Système selon la revendication 1, comprenant en outre une administration par intraveineuse des ferrofluides au flux sanguin.

22. Système selon la revendication 21, dans lequel les ferrofluides sont concentrés sur un site de la plaque accumulée (2) avant l'enlèvement de ladite plaque accumulée (2) par lesdits ferrofluides.

23. Système selon la revendication 1, dans lequel les ferrofluides sont disposées de façon à rompre et enlever la plaque accumulée (2) et polir une surface intérieure du vaisseau sanguin.

24. Système selon la revendication 11, dans lequel le guide-fil (400) avec la pointe magnétique (410) comprend en outre un dispositif de recapture magnétique pour re-capturer et diriger les composants magnétiques (100) dans les ferrofluides à travers le cathéter ou micro-cathéter pour enlever les composants magnétiques (100) des ferrofluides du vaisseau sanguin (1).
